# EUROPEAN PATENT SPECIFICATION

(11) **EP 3 701 956 B1**
(45) Date of publication and mention of the grant of the patent: **26.06.2024**
(21) Application number: 18870872.1
(22) Date of filing: 14.05.2018
(51) Int. Cl.: A61K 33/00, A61P 25/28, A61K 9/00

(54) **PROPHYLACTIC AND/OR THERAPEUTIC AGENT FOR DEMENTIA**
PROPHYLAKTISCHES UND/ODER THERAPEUTISCHES MITTEL BEI DEMENZ
AGENT PROPHYLACTIQUE ET/OU THÉRAPEUTIQUE CONTRE LA DÉMENCE

(30) Priority: 27.10.2017 JP 2017208376
(43) Date of publication of application: 02.09.2020
(73) Proprietor: Mitos Co., Ltd., Kawasaki-shi, Kanagawa 211-0005 (JP); ONO, Hirohisa, Oooka, Numazu-shi Shizuoka 4100022 (JP); Nishijima, Yoji, Oooka Numazu-shi Shizuoka 4100022 (JP)
(72) Inventor: ONO, Hirohisa, Numazu-shi Shizuoka 4100022 (JP); NISHIJIMA, Yoji, Numazu-shi Shizuoka 4100022 (JP); OHTA, Shigeo, Kawasaki-shi Kanagawa 211-0004 (JP); ASADA, Takashi, Tokyo 113-0034 (JP)
(74) Representative: Mewburn Ellis LLP
(86) International application number: PCT/JP2018/018483
(87) International publication number: WO 2019/082428

(56) References cited:
- EP-A1- 1 946 762
- EP-B1- 1 946 762
- JP-A- 2009 114 084
- JP-A- 2009 114 084
- JP-A- 2015 183 005
- JP-A- 2018 030 804
- US-A1- 2015 272 867
- CAI WANG ET AL: "Hydrogen-rich saline reduces oxidative stress and inflammation by inhibit of JNK and NF-B activation in a rat model of amyloid-beta-induced Alzheimer's disease", NEUROSCIENCE LETTERS, ELSEVIER, AMSTERDAM, NL, vol. 491, no. 2, 7 January 2011 (2011-01-07), pages 127-132, XP028145405, ISSN: 0304-3940, DOI: 10.1016/J.NEULET.2011.01.022 [retrieved on 2011-01-14]
- LI J ET AL: "Hydrogen-rich saline improves memory function in a rat model of amyloid-beta-induced Alzheimer's disease by reduction of oxidative stress", BRAIN RESEARCH, ELSEVIER, AMSTERDAM, NL, vol. 1328, 30 April 2010 (2010-04-30), pages 152-161, XP026999980, ISSN: 0006-8993 [retrieved on 2010-02-19]
- RYUJI YONAMINE ET AL: "Coadministration of Hydrogen Gas as Part of the Carrier Gas Mixture Suppresses Neuronal Apoptosis and Subsequent Behavioral Deficits Caused by Neonatal Exposure to Sevoflurane in Mice", ANESTHESIOLOGY, vol. 118, no. 1, 1 January 2013 (2013-01-01), pages 105-113, XP055178856, ISSN: 0003-3022, DOI: 10.1097/ALN.0b013e318275146d
- KIYOMI NISHIMAKI ET AL: "Effects of Molecular Hydrogen Assessed by an Animal Model and a Randomized Clinical Study on Mild Cognitive Impairment", CURRENT ALZHEIMER RESEARCH, vol. 15, no. 5, 14 March 2018 (2018-03-14) , pages 482-492, XP055661340, NL ISSN: 1567-2050, DOI: 10.2174/1567205014666171106145017
- KYOTA FUJITA ET AL: "Therapeutic Approach to Neurodegenerative Diseases by Medical Gases: Focusing on Redox Signaling and Related Antioxidant Enzymes", OXIDATIVE MEDICINE AND CELLULAR LONGEVITY, vol. 2012, 1 January 2012 (2012-01-01), pages 1-9, XP055631635, US ISSN: 1942-0900, DOI: 10.1155/2012/324256
- CHENG, LIN-LIU: "Hydrogen therapy: from mechanism to cerebral diseases", Medical Gas Research, vol. 6, no. 1, March 2016 (2016-03), pages 48-54, XP055596903,
- OHTA, SHIGEO et al.: "Recent progress toward hydrogen medicine: potential of molecular hydrogen for preventive and therapeutic applications", Current Pharmaceutical Design, vol. 17, 2011, pages 2241-2252, XP055535704, DOI: 10.2174/138161211797052664
- Shigeo Ota: "5. Ninshish no yob [Prevention of dementia]", Newsletter, vol. 1 2010, pages 1-4, XP009520108, Retrieved from the Internet: URL:http://www2.nms.ac.jp/ig/soudan/hpb/pd f/Newsletter1012.pdf
- Cheng-Lin Liu ET AL: "Hydrogen therapy: from mechanism to cerebral diseases", Medical Gas Research, vol. 6, no. 1, 1 March 2016 (2016-03-01), pages 48-54, XP055596903, London, UK ISSN: 2045-9912, DOI: 10.4103/2045-9912.179346

## Description

### Technical Field

The present invention relates to a therapeutic agent for dementia.

### Background Art

Examples of therapeutic agents for dementia, particularly Alzheimer-type dementia include acetylcholinesterase inhibitors and N-methyl-D-aspartate (NMDA) receptor antagonists, and these pharmaceutical agents only temporarily improve the neural functions, and do not provide radical therapy. Thus, in the moderate or more severe stage, these agents become less effective, and the cognitive function declines more rapidly. In this stage, deterioration of the cognitive function cannot be addressed by existing treatment methods, and the patients tend to be given treatment as severe dementia in hospitals or care facilities.

Meanwhile, hydrogen has recently been found to have various actions besides the conventional antioxidant capacity and many additional reports have already been published also on its effect and safety (Patent Literature 1). Patent Literature 2 provides an inhalation-type pharmaceutical composition for Alzheimer's disease and preparation method thereof, comprising a first gas an atomized medicine. The first gas comprises hydrogen. The gas volume concentration of hydrogen in the inhalation-type pharmaceutical composition is between 2 to 96%. The atomized medicine is selected from a group comprising rivastigmine hydrogen tartrate, donepezil hydrochloride, galantamine hydrobromide, memantine and any combination thereof.

Non-Patent Literature 1 reports that hydrogen-rich saline reduces oxidative stress and inflammation by inhibition of JNK and NF-kB activation in a rat model of amyloid-beta-induced Alzheimer's disease. Non-Patent Literature 2 reports that hydrogen-rich saline improves memory function in a rat model of amyloid-beta-induced Alzheimer's disease by reduction of oxidative stress.

### Citation List

### Patent Literature

Patent Literature 1: International Publication No. WO 2007/021034 (also published as EP 1 946 762)
Patent Literature 2: US Patent Application Publication No. US 2015/0272867

### Non-Patent Literature

Non-Patent Literature 1: Wang, C. et al., Neuroscience Letters 2011; 491:127-132
Non-Patent Literature 2: Li, J. et al., Brain Research 2010; 1328:152-161

### Summary of Invention

### Technical Problem

An object of the present invention is to provide a therapeutic agent for dementia.

### Solution to Problem

The present inventors have made a patient inhale hydrogen gas to administer a larger amount of hydrogen, and resultantly successfully treated moderate and severe dementia, leading to completion of the present invention.

The present invention provides hydrogen gas for use in the treatment of Alzheimer-type dementia in a patient in whom a therapeutic effect of cholinesterase inhibitors and/or N-methyl-D-aspartate (NMDA) receptor antagonists can no longer be exhibited, as defined in the claims.

The description of the present application refers to the disclosure of JP Patent Application No. 2017-208376, which the priority of the present application is based on. Advantageous Effects of Invention

According to the present invention, it is possible to treat dementia.

### Brief Description of Drawings

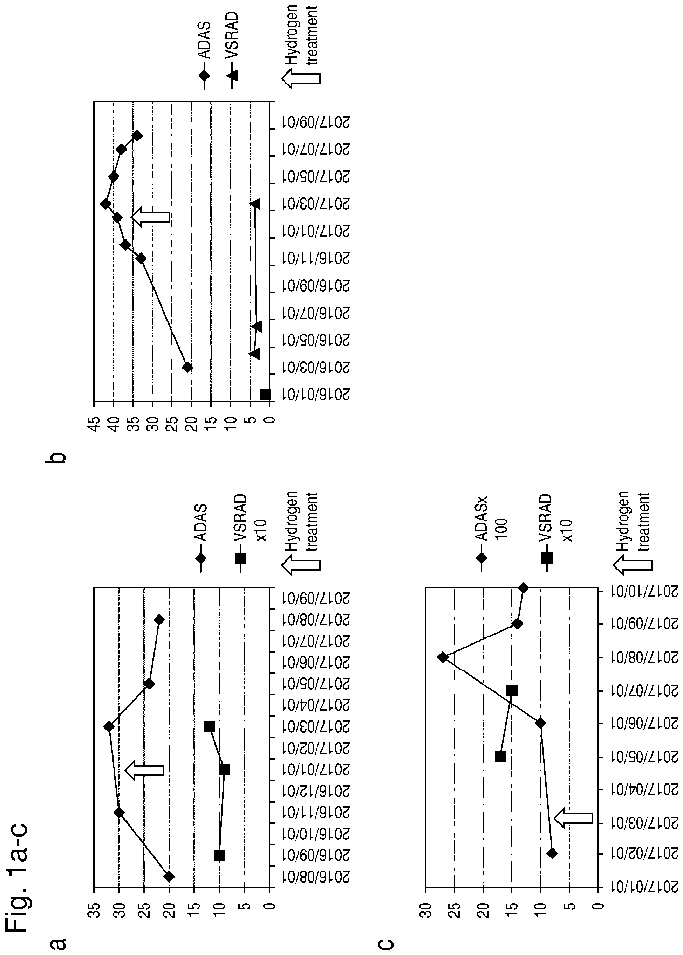

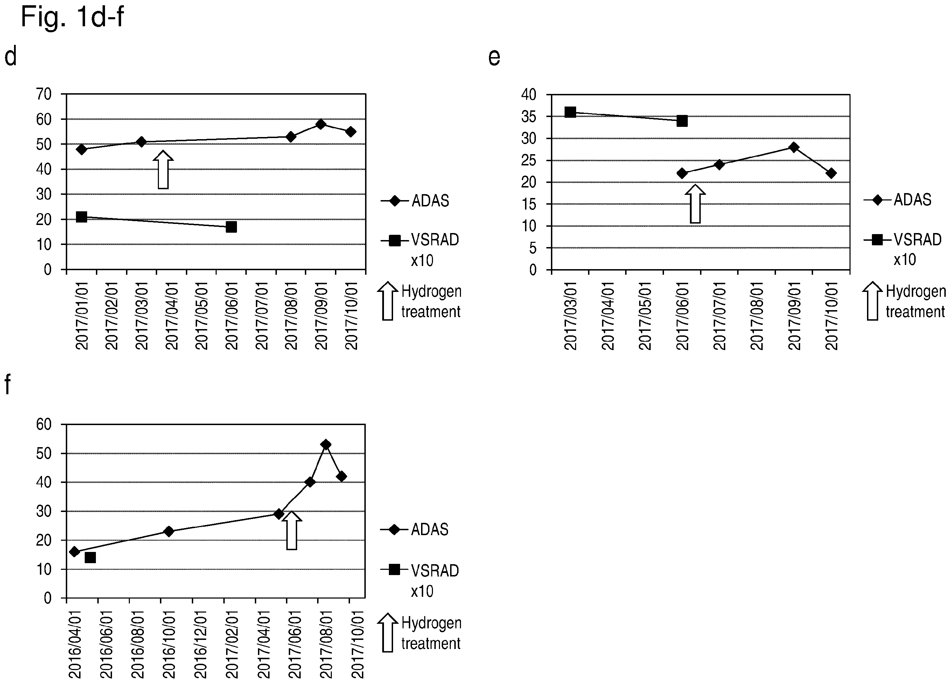

[Figures 1a to 1c] Figures 1a to 1c show changes in ADAS-cog and VSRAD in a treatment group (six patients) after completion of a 6-month treatment period (a-c). ADAS is a cognitive function test, in which it is considered that the symptom is improved when the score decreases. VSRAD is image inspection, in which the degrees of brain shrinkage are examined by MRI, compared between contemporaries, and scored. The arrow in each figure indicates a time at which hydrogen treatment is started.
[Figures 1d to 1f] Figures 1d to 1f show changes in ADAS-cog and VSRAD in a treatment group (six patients) after completion of a 6-month treatment period (d-f). ADAS-cog represents is a cognitive function test, in which it is considered that the symptom is improved when the score decreases. VSRAD is image inspection, in which the degrees of brain shrinkage are examined by MRI, compared between contemporaries, and scored. The arrow in each figure indicates a time at which hydrogen treatment is started.

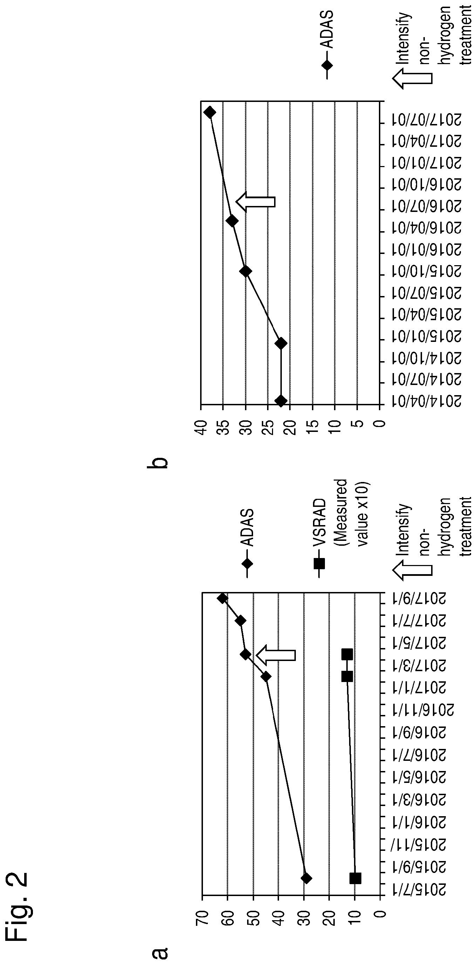

[Figure 2] Figure 2 shows changes in ADAS-cog and VSRAD of control cases (two patients) after completion of a 6-month observation period. The arrow in each figure indicates a time at which the amount of an antidementia agent is increased to the maximum, if it is not the maximum, and non-hydrogen treatment that is acceleration of rehabilitation (mainly improvement of walking by use of parallel bars or with the aid of a silver car, training on toilet use, or the like) is intensified.

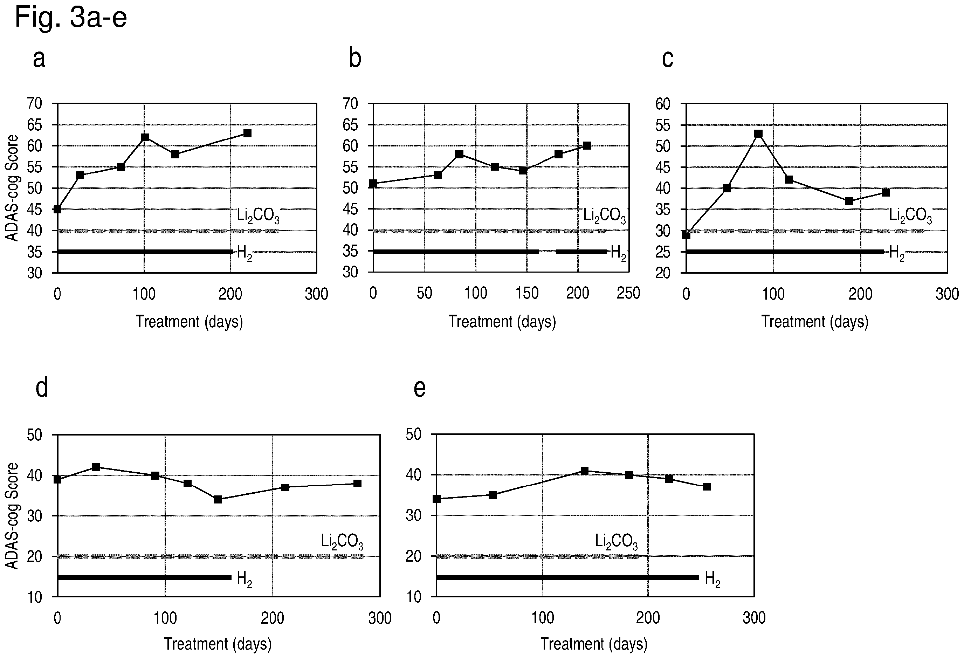

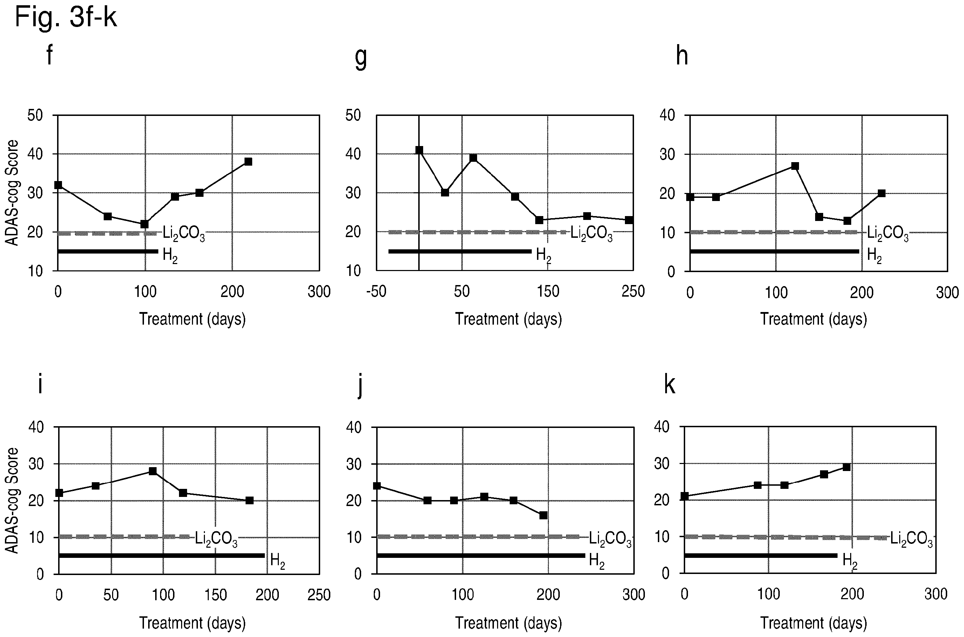

[Figures 3a to 3e] Figures 3a to 3e are diagrams showing the results of analyzing ADAS-cog scores of 11 dementia patients given H₂ gas (first). Figures 3a to 3e show time-dependent changes of ADAS-cog scores of patients a to e. The straight line and the dotted line represent the periods of Hz treatment and Li₂CO₃ treatment, respectively. In the Hz treatment of patient b, treatment was temporarily stopped, and then resumed, and therefore an effect at the time of completion of first H₂ treatment was taken into consideration.
[Figures 3f to 3k] Figures 3f to 3k are diagrams showing the results of analyzing ADAS-cog scores of 11 dementia patients given H₂ gas (f-k). Figures 3f to 3k show time-dependent changes of ADAS-cog scores of patients f to k. The straight line and the dotted line represent the periods of H₂ treatment and Li₂CO₃ treatment, respectively.

### Description of Embodiments

The present invention will be described in detail below.

The present invention provides hydrogen gas for use in the treatment of Alzheimer-type dementia, as defined in the claims, which is also referred to as "the therapeutic agent" of the present invention.

By the therapeutic agent of the present invention, cognitive functions in human moderate and severe dementia can be improved, and progress into dementia can be prevented or retarded.

The dementia is a symptom or condition caused by disintegration of nerve cells in the brain due to a disease. Progress of dementia deteriorates comprehension and judgment, resulting in disturbance of social life and everyday life. The dementia includes Alzheimer-type dementia (Alzheimer's disease, as claimed), dementia with Lewy bodies (not explicitly claimed), and vascular dementia (not explicitly claimed).

The therapeutic agent of the present invention is used so as to make a human inhale hydrogen gas at 1 to 18.5% (v/v) for 1 hour two or more times a day. It is effective to use the therapeutic agent so as to make a human inhale hydrogen gas at 3 to 4% (v/v) for 1 hour two times a day.

The flow rate of hydrogen gas inhaled may be 4 to 6 liters/min, and is preferably 6 to 8 liters/min for patients having hyperpnea.

In administration of hydrogen gas, a patient may be made to wear an aspiration mask and inhale the hydrogen gas through the mouth and the nose. Alternatively, a patient may enter a hermetically sealed space (e.g. hermetically sealed chamber or compartment) supplied with hydrogen gas, and breathe there to inhale the hydrogen gas.

Preferably, hydrogen gas is inhaled with air and/or oxygen gas added to the hydrogen gas. Addition of oxygen gas reduces the side effect of the preventive and/or therapeutic agent of the present invention, leading to enhancement of safety. The oxygen gas may be added in an amount about 1/2 times the amount of the hydrogen gas. The hydrogen gas may be mixed with gas other than air and oxygen gas (e.g. inert gas such as nitrogen gas, helium gas or argon gas, or anesthetic gas such as laughter gas).

Also disclosed but not explicitly claimed is an apparatus for preventing and/or treating dementia, comprising a container comprising hydrogen gas, a gas aspiration unit, and a pipe for supplying the gas in the container to the gas aspiration unit. The container is, for example, a hydrogen gas cylinder. The gas aspiration unit is, for example, an aspiration mask, or a hermetically sealed chamber or compartment. The apparatus may further comprise a container comprising gas such as oxygen gas, inert gas, air or anesthetic gas, and in this case, the hydrogen gas and the other gas (oxygen gas, inert gas, air, anesthetic gas or the like) may be supplied to the gas aspiration unit separately or after being mixed. For example, a gas aspiration bag containing hydrogen gas may be directly connected to the aspiration mask, and supplied with hydrogen gas from a gas cylinder containing hydrogen gas. The gas is supplied to the gas aspiration unit through a pipe, and administered to a patient. Apparatuses comprising a container containing hydrogen gas, a gas aspiration unit, and a pipe for supplying the gas in the container to the gas aspiration unit are described in JP Patent No. 5091364, No. 5100911, No. 5900688 and the like, and these apparatuses or modifications thereof can be used.

Administration of hydrogen gas significantly decreases the ADAS-cog (Alzheimer's Disease Assessment Scale-cognitive subscale) score. The ADAS-cog, which is a method for evaluating the cognitive function, evaluates 11 items: Word Recall Task, Naming Objects and Fingers, Following Commands, Constructional Praxis, Ideational Praxis, Orientation, Word Recognition Task, Remembering Test Directions, Spoken Language, Comprehension, Word-Finding Difficulty. The score ranges from 0 to 70 points, and the higher the score, the more severe the Alzheimer-type dementia. Donepezil (trade name: Aricept) pharmaceutically approved as a therapeutic agent for Alzheimer's disease has been reported to decrease the ADAS-cog score by 2 to 3 points after 2 to 3 months after administration, and when treatment decreases the ADAS-cog score by 3 or more points, it is determined that the treatment is useful for improvement of the cognitive function.

When the therapeutic agent for dementia according to the present invention, comprising hydrogen gas as an active ingredient, is administered over 3 to 12 months, for example 6 months, the ADAS-cog score can be decreased by at least 2 points, preferably at least 3 points, more preferably 3.4 points, from that at the start of administration. The ADAS-cog score can be decreased by at least 4 points, preferably at least 5 points, more preferably at least 6 points, especially preferably 8.5 points, from the worst score due to a time-dependent change during the treatment period.

An improving effect on dementia can be obtained in at least 70%, preferably at least 80%, more preferably at least 90% of patients given the therapeutic agent for dementia according to the present invention, comprising hydrogen gas as an active ingredient.

Inhalation of hydrogen gas exhibits an improving effect for Alzheimer-type dementia patients in whom a therapeutic effect of acetylcholinesterase inhibitors and/or N-methyl-D-aspartate (NMDA) receptor antagonists pharmaceutically approved as therapeutic agents for Alzheimer-type dementia can no longer be exhibited. Therefore, hydrogen gas exhibits an effect through an action mechanism different from that of the therapeutic effect of cholinesterase inhibitors and/or N-methyl-D-aspartate (NMDA) receptor antagonists.

### Examples

The present invention will be described in further detail below with reference to the Examples. The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy.

### [Example 1]

### Subject cases

Patients (outpatients in Iryohojin Shadanshinwakai Nishijima Byoin (Numazu-shi, Shizuoka)) in whom rapid deterioration (deterioration by 3 or more points over 1 year) in a cognitive function test such as MMSE or ADAS-cog occured although existing four agents for dementia were used over a long period of time, and improvement is not attained by other ordinary treatment methods. Combined use of antihypertensive drugs, antilipemic drugs, therapeutic agents for heart disease, anticoagulants, antiplatelet preparations, psychotropic agents, liver supporting agents, renal agents, various nutritional supplements, all government-approved antidementia agents and the like which have been heretofore administered is not limited.

### Inapplicable cases

Patients for whom it is considered difficult to repeatedly inhale hydrogen gas over a long period of time because of severe medical disease, chronic chest disease (chronic obstructive pulmonary disease (COPD), asthma or the like), mouth inflammation or the like.

### Treatment method

Hydrogen gas at about 3 to 4% is inhaled through a normal oxygen inhalation mask or the like for 1 hour twice a day (morning and evening) by normal breathing. Safety of long-time inhalation of air incorporating only hydrogen is also examined, and oxygen is added in an amount about half (1/2 times) the amount of hydrogen, so that development of hypoxemia due to inhalation of only mixed gas of hydrogen and air is suppressed to enhance safety of inhalation of hydrogen gas. Also, the amount of active oxygen is minimized which is generated in supply of an excessive amount of oxygen to involved tissues (e.g. brain infarction and cardiac infarction lesions, and dementia diseased tissues) that cannot utilize oxygen due to existing tissue damage. There have been reported many cases where hydrogen generally reduces the amount of such oxidizing radicals, and hydrogen does not react with oxygen molecules in the absence of a catalyst.

### Evaluation of clinical effect

### Test for dementia by face-to-face questioning

### a. Effective cases

Patients for whom it is determined from a score in the ADAS-cog cognitive function test that a therapeutic effect is achieved.
(1) Patients in whom the tendency of deterioration in the ADAS score starting before treatment continued in the early stage during the treatment period, but the score was lower at the time of completion of treatment than at the start of treatment (= clinical improvement) (Patients with improvement after temporary deterioration).
(2) Patients in whom deterioration in ADAS-cog score did not occur by completion of the treatment period (= about 6 months later) (non-deterioration patients).

### b. Ineffective cases

Patients in whom temporary short-term improvement in the score in the ADAS cognitive function test might be attained before completion of treatment, but the score was worse at the time of completion than at the start of treatment.

### Results of treatment

### A. Treatment group (six patients)

Among the patients for whom the 6-month treatment period was completed, the treatment was effective in all of the six patients. (1)-six patients. (2)-zero patients. The patients are shown in Figures 1a to 1c and Figures 1d to 1f.

### B. Control cases (two patients)

The tendency of deterioration continued in two patients for whom the 6-month observation period was completed. For these patients, the amount of an antidementia agent was increased to the maximum if it was not the maximum, and non-hydrogen treatment for acceleration of rehabilitation (mainly improvement of walking by use of parallel bars or with the aid of a silver car, training on toilet use, or the like) was intensified. The patients are shown in Figure 2.

### Discussion

Dementia spreads all over the world. In Japan alone, the number of dementia patients is about 4,000,000, and will increase to about 7,000,000 6 years later, so that the morbidity of elderly people aged 65 years or older will exceed 20%. To date, 200 or more therapeutic agents for dementia have been developed by many pharmaceutical companies, and some of them have been ballyhooed and considered very promising, and highly expected by medical experts, but at present, pharmaceutical agents evaluated as being effective do not exist at all. There are many cases where a pharmaceutical agent is expected to have an effect in animal experiments, but there is no effect in clinical trials. It should be emphasized that the effect can be verified only by conducting clinical trials with patients. At present, there are four government-approved pharmaceutical agents for dementia, and all the agents only ensure that in a cognitive function test called MMSE (perfect score indicating a normal state is 30 points), improvement of the score by at most about 2 or 3 points occurs over about 1 year. Thereafter, not only the effect cannot be obtained, but also deterioration is accelerated to the extent that the deterioration speed becomes higher than the deterioration speed in a patient who has not taken the antidementia agent for some reason. The reason for this may be as follows. The current pharmaceutical agents are acetylcholinesterase inhibitors, in principle, and are intended to suppress decomposition of acetylcholine which is a transmitter at a synapse being a site of cerebral nerve stimulus transmission, and thus artificially increase the concentration of acetylcholine to improve the cognitive function. Accordingly, expression or the like of genes that produce acetylcholine originally present in the brain declines, and resultantly, besides production and accumulation of brain tissue damaging toxic substances due to, for example, abnormal perphosphorylation of nerve cells, which is an original cause of dementia, an iatrogenic pathological condition is produced, so that dementia is rather aggravated. The N-methyl-D-aspartate (NMDA) receptor antagonists having another action mechanism are pharmaceutical agents which suppress excessive stimulation to protect nerve cells. However, many side effects have been found (reference: Yu Nakamura, Japanese Journal of Biological Psychiatry, 22(4), 227-235). Thus, we searched for a substance which may have a mechanism of action on sites entirely different from those for the acetylcholinesterase systems etc., and is quite safe for elderly people and easy to administer, and which serves as a pharmaceutical agent having multiple actions and diffuses in the human body without being hindered by barriers, hurdles and the like in contrast to conventional pharmaceutical agents developed with dementia set as a single target, leading to a series of failures. As a result, lithium and hydrogen were found to be candidates for such a substance. Lithium has been already used for dementia abroad, and shown to be effective to a limited extent, but has the disadvantage of generating side effects on which PMDA issued a warning particularly for elderly people. On the other hand, hydrogen has been confirmed to have a high level of safety, and shown to have an effect on a variety of cell signals and genes probably related to causes of dementia and on various factors related to production and removal of various toxic substances in the brain in dementia. However, there has been no report of treatment performed with hydrogen yet in patients with severe dementia clinically. One of the causes for this was thought to be that the fundamental mechanism of action of hydrogen, which makes hydrogen effective for a wide range of diseases as described above, cannot be explained only with the conventional neutralizing action on oxidizing radicals and free radical chain reaction preventive capacity, and further, there is a concern that hydrogen is insufficient for coping with difficult problems at a time such as correction of protein misfolding, removal of toxic substances deposited in the brain, and regeneration of brain tissues destroyed and shrunk by the toxic substances, which are necessary for treatment of dementia. Thus, for the first time in the world, we performed treatment through inhalation of hydrogen gas in relatively severe dementia patients, for whom conventional antidementia agents were no longer effective and dementia was aggravated, and consequently good results were obtained. An inhalation method was used in consideration of the following: hydrogen is discharged from the lungs before reaching the brain in hydrogen treatment using a conventional method such as drinking of hydrogen water or intravenous administration, and a large amount of hydrogen is needed for treatment of brain disease, particularly for treatment of dementia requiring actions on a wide range of regions. Further, it was required that during the inhalation time, the patients be accompanied by their family members, who frequently check the position of a mask, and the like. Patients whose family members were unable to meet the requirement were excluded from the clinical trial.

### [Example 2]

### Selection of patients

Patients were selected on the basis of the following three criteria.
(1) Deterioration in the score occurs such that the ADAS-cog score based on formula [70 - (MMSE × 2.33)] (J. Caro et al., BMC Neurol 2002;2:6.) or the ADAS-cog score calculated in terms of MMSE (Mini-Mental State Examination) exceeds 10.
(2) The patient has been diagnosed with Alzheimer-type dementia, and already treated with an acetylcholinesterase inhibitor or a N-methyl-D-aspartate (NMDA) receptor antagonist, but further deterioration in the ADAS-cog score occurs.
(3) The patient does not have a serious airway disease which may hinder adequate inhalation of H₂, such as COPD (chronic obstructive pulmonary disease), pneumonia, bronchitis or asthma.

Before the test, the patient was given lithium carbonate (Li₂CO₃) at 400 mg/day over 1 week to confirm that kidney and liver functions were within normal ranges.

Finally, 11 patients were selected, and treated with H₂ gas.

### Treatment

Since the probability of suffering complications increases as the concentration of lithium in blood increases, the dosage of lithium carbonate was reduced (W.S. Waring, Toxicol Rev 2006;25:221-230.). The patients of the treatment group were given oral administration of a 200 mg tablet of lithium carbonate (Li₂CO₃) (Mitsubishi Tanabe Pharma Corporation) twice a day and inhalation of 3% H₂ twice a day. The test period was 6 months. As the gas containing H₂, H₂ gas (3%) containing 21% oxygen was generated using a portable H₂ generator (Nishijima/Ecore Gas Hydrogen Generator) (H. Ono et al., J Stroke Cerebrovasc Dis 2017;26:2587-2594.). As reported previously, the blood concentration was confirmed to increase (H. Ono et al., J Stroke Cerebrovasc Dis 2017;26:2587-2594.). Since lithium carbonate is used as a therapeutic agent for depression and mania (bipolar disorder), and it was reported that patients who ingested lithium carbonate less likely developed dementia, lithium carbonate was used in this Example.

### Evaluation

The patients were evaluated by a blind method using the ADAS-cog score.

The ADAS-cog test was conducted every 1 to 2 months. The efficacy of treatment with respect to the ADAS-cog score was determined by comparing the ADAS-cog score at the start of the test with two or three tests during treatment.

### Results of treatment

The ADAS-cog scores of most of the patients were improved by the H₂ therapy.

Figures 3a to 3e and Figures 3f to 3k show profiles of changes of ADAS-cog scores of the patients subjected to the H₂ therapy and the lithium therapy in combination. Diagrams a to k show the respective results for 11 patients. In patients corresponding to f and h, deterioration in the score occurred soon after completion of the combination therapy. On the other hand, in patients corresponding to e and i, improvement by H₂ occurred even after addition of lithium was stopped. In patients corresponding to a, b, c, d, g and h, deterioration (continuous increase) in the score occurred after H₂ treatment was stopped. Therefore, it was revealed that the primary effect of the combination therapy resulted only from inhalation of H₂.

At the end of the H₂ treatment period, the score of each of seven patients (c, d, f, g, h, i and j) was improved (decreased) from that at the start time. The score at the time of completion of Hz treatment was not as good as the initial level, and in three patients (a, b and e), deterioration in the score occurred once, followed by slight improvement in the score. One patient (k) was unresponsive. Table 1 shows the results of analyzing the ADAS-cog scores at the start of administration of hydrogen gas, after deterioration and after completion of administration of hydrogen gas in 11 dementia patients given H₂ gas. As shown in Table 1, the average of the scores including the score of the unresponsive patient was -3.4 (with respect to the initial score) or -8.5 (with respect to the worst score) at the time of completion of H₂ treatment.

**[Table 1]**

| Patient | Sex | Age | EKG | Creatine (mg/dl) | LivEnzymes | Diabetes | Lipidemia | Initial score: A | Worst score: B | Final score: C | C-A | C-B |
|---|---|---|---|---|---|---|---|---|---|---|---|---|
| **a** | F | 68 | T-wave | 0.6 | N | N | Moderate | 53 | 62 | 58 | 5 | -4 |
| **b** | F | 73 | N | 0.8 | N | N | Moderate | 51 | 58 | 54 | 3 | -4 |
| **c** | F | 85 | r-BBB | 0.7 | N | Mild | N | 40 | 53 | 37 | -3 | -16 |
| **d** | F | 83 | T-wave | 0.5 | N | N | N | 39 | 42 | 34 | -5 | -8 |
| **e** | M | 84 | N | 0.6 | N | N | N | 35 | 41 | 37 | 2 | -4 |
| **f** | F | 77 | N | 0.8 | N | N | Mild | 30 | 30 | 22 | -8 | -8 |
| **g** | F | 80 | N | 0.7 | N | N | N | 41 | 41 | 23 | -18 | -18 |
| **h** | F | 70 | r-BBB | 0.5 | Mild abnormal | N | Moderate | 19 | 27 | 13 | -6 | -14 |
| **i** | F | 85 | Bordarlina | 0.8 | N | N | N | 22 | 29 | 20 | -2 | -9 |
| **j** | F | 71 | N | 0.6 | N | N | N | 24 | 24 | 16 | -8 | -8 |
| **k** | F | 70 | N | 0.7 | N | N | N | 21 | 24 | 24 | 3 | 0 |
| Average | | 76.9 | | | | | | 34.1 | 39.2 | 30.7 | | |
| S.D. (±) | | 6.42 | | | | | | 11.3 | 13.1 | 14.1 | 6.4 | 5.3 |

| | | | | | | | | | | | | |
|---|---|---|---|---|---|---|---|---|---|---|---|---|
| C shows an ADAS-cog score at the time of completion of treatment by inhalation of H₂. | | | | | | | | | | | | |

In five controls, improvement by treatment did not occur. In a hospital at which the test was conducted, about 200 patients were examined, and there was not an Alzheimer-type dementia patient whose ADAS-cog score was improved within 6 months when H₂ treatment was not performed. Therefore, H₂ treatment provided significant improvement in the ADAS-cog score.

### Discussion

In the combination therapy with H₂ and lithium, improvement in the ADAS-cog score occurred in 10 out of 11 Alzheimer-type dementia patients. The ADAS-cog score (W.G. Rosen et al., Am J Psychiatry 1984;141:1356-1364.) is a general cognitive function index which is most widely used in clinical trials of AD, and the ADAS-cog score covers a plurality of cognitive areas including memory, language, praxis and act of orienting. In the combination therapy according to the present invention, there were three possibilities. The first possibility is that combination of H₂ with lithium was essential for synergic effect. The second possibility is that mainly lithium was effective, and H₂ reduced side effects of lithium or reinforced the efficacy of lithium. The third possibility is that Hz alone was effective for treatment.

The results of this Example show that the therapeutic effect resulted only from inhalation of H₂ because improvement in the score with H₂ continued even when lithium was not present (e and i). In contrast, when H₂ treatment was stopped, deterioration (continuous increase) in the score occurred in patients corresponding to a, b, c, d, g and h. That is, when H₂ was not used in combination with lithium, i.e., lithium was used alone, the cognitive function was not improved, and thus only H₂ improved the cognitive function.

The improvement by inhalation of H₂ is significant when compared to the effect of donepezil which is used for temporary improvement of the dementia symptom of Alzheimer-type dementia. Donepezil decreased the ADAS-cog score by 3 points (-3) after 6 weeks. However, after 6 months, deterioration occurred in the placebo group, and the score of the donepezil administration group turned back to the initial value (K.R. Krishnan et al., Am J psychiatry 2003;160:2003-2011.). In H₂ treatment in this Example, the average of changes of scores of subjects including unresponsive subjects was -3.4 with respect to the initial stage. Further, the average of changes of scores from the worst score to the score at the end of H₂ treatment was -8.5. That is, the effect of H₂ gas was obtained with a high ratio of 10 out of the 11 patients, and H₂ gas had a significantly higher therapeutic effect as compared to donepezil.

Further, the patients in this Example started hydrogen gas inhalation treatment after elimination of the therapeutic effect of the acetylcholinesterase inhibitor and/or N-methyl-D-aspartate (NMDA) receptor antagonist pharmaceutically approved as a therapeutic agent for Alzheimer-type dementia. Therefore, it is evident that the improvement effect of inhalation of hydrogen gas was exhibited through an action mechanism different from that of the effect of the pharmaceutically approved pharmaceutical agents. The present invention can also be applied after conventional pharmaceutically approved agents no longer exhibit a therapeutic effect, and thus the present invention is extremely useful.

### Industrial Applicability

The present invention can be utilized for treatment of dementia.

## Claims

1. Hydrogen gas for use in the treatment of Alzheimer-type dementia in a patient in whom a therapeutic effect of cholinesterase inhibitors and/or N-methyl-D-aspartate (NMDA) receptor antagonists can no longer be exhibited, by human inhalation of the hydrogen gas at 1 to 18.5% (v/v) for 1 hour two or more times a day.

2. Hydrogen gas for use according to claim 1, wherein said use is with air and/or oxygen gas added to the hydrogen gas.

3. Hydrogen gas for use according to claim 2, wherein the oxygen gas is added in an amount about 1/2 times the amount of the hydrogen gas.

4. Hydrogen gas for use according to any one of claims 1 to 3, wherein said use is to decrease an ADAS-cog score by at least 3 points from that at the start of treatment when administered over 3 to 12 months.

5. Hydrogen gas for use according to any one of claims 1 to 4, wherein said use is to decrease the ADAS-cog score by at least 4 points from that at the time of the worst condition due to a time-dependent change when administered over 3 to 12 months.

6. Hydrogen gas for use according to any one of claims 1 to 5, wherein said treatment is by an action mechanism which is different from cholinesterase inhibitors and/or N-methyl-D-aspartate (NMDA) receptor antagonists.

## Patentansprüche

1. Wasserstoffgas zur Verwendung bei der Behandlung von Demenz vom Alzheimer-Typ bei einem Patienten, bei dem kein therapeutischer Effekt von Cholinesterasehemmern und/oder N-Methyl-D-aspartat- (NMDA-) Rezeptorantagonisten mehr eintritt, durch einstündiges menschliches Inhalieren des Wasserstoffgases bei 1 bis 18,5 Vol.-% zwei oder mehrere Male pro Tag.

2. Wasserstoffgas zur Verwendung nach Anspruch 1, wobei die Verwendung mit Luft und/oder Sauerstoffgas erfolgt, die/das dem Wasserstoffgas zugesetzt ist.

3. Wasserstoffgas zur Verwendung nach Anspruch 2, wobei das Sauerstoffgas in einer Menge zugesetzt ist, die etwa 1/2-mal der Menge des Wasserstoffgases entspricht.

4. Wasserstoffgas zur Verwendung nach einem der Ansprüche 1 bis 3, wobei die Verwendung der Senkung einer ADAS-cog-Zahl um mindestens drei Punkte in Bezug auf die Zahl, die zu Beginn der Behandlung verzeichnet wurde, bei einer Verabreichungsdauer von 3 bis 12 Monaten dient.

5. Wasserstoffgas zur Verwendung nach einem der Ansprüche 1 bis 4, wobei die Verwendung der Senkung der ADAS-cog-Zahl um mindestens vier Punkte in Bezug auf die Zahl, die zum Zeitpunkt des schlechtesten Zustands aufgrund einer zeitabhängigen Veränderung verzeichnet wurde, bei einer Verabreichungsdauer von 3 bis 12 Monaten dient.

6. Wasserstoffgas zur Verwendung nach einem der Ansprüche 1 bis 5, wobei die Behandlung durch einen Wirkungsmechanismus erfolgt, der sich von Cholinesterasehemmern und/oder N-Methyl-D-aspartat- (NMDA-) Rezeptorantagonisten unterscheidet.

## Revendications

1. Hydrogène gazeux à utiliser dans le traitement de la démence de type Alzheimer chez un patient chez lequel un effet thérapeutique d'inhibiteurs de cholinestérase et/ou d'antagonistes de récepteur de N-méthyl-D-aspartate (NMDA) ne peut plus être démontré, par inhalation humaine de l'hydrogène gazeux de 1 à 18,5 % (v/v) pendant 1 heure deux fois ou plus par jour.

2. Hydrogène gazeux à utiliser selon la revendication 1, dans lequel ladite utilisation est avec de l'air et/ou de l'oxygène gazeux ajouté à l'hydrogène gazeux.

3. Hydrogène gazeux à utiliser selon la revendication 2, dans lequel l'oxygène gazeux est ajouté en une quantité d'environ 1/2 fois la quantité de l'hydrogène gazeux.

4. Hydrogène gazeux à utiliser selon l'une quelconque des revendications 1 à 3, dans lequel ladite utilisation consiste à diminuer un score ADAS-cog d'au moins 3 points par rapport à celui au début du traitement lorsqu'il est administré sur 3 à 12 mois.

5. Hydrogène gazeux à utiliser selon l'une quelconque des revendications 1 à 4, dans lequel ladite utilisation consiste à diminuer le score ADAS-cog d'au moins 4 points par rapport à celui au moment de la pire condition due à un changement dépendant du temps lorsqu'il est administré sur 3 à 12 mois.

6. Hydrogène gazeux à utiliser selon l'une quelconque des revendications 1 à 5, dans lequel ledit traitement est effectué par un mécanisme d'action qui est différent d'inhibiteurs de cholinestérase et/ou d'antagonistes de récepteur de N-méthyl-D-aspartate (NMDA).
